**Europäisches Patentamt**

(19) **European Patent Office**

**Office européen des brevets**

(11) Veröffentlichungsnummer: **0 045 006**
**B1**

(12) # EUROPÄISCHE PATENTSCHRIFT

(45) Veröffentlichungstag der Patentschrift:
18.04.84

(51) Int. Cl.³: **A 61 K 31/655,** C 07 C 107/06

(21) Anmeldenummer: **81105533.4**

(22) Anmeldetag: **15.07.81**

(54) **Mittel zur Behandlung von Colitis ulcerosa, Enteritis regionalis Crohn (Morbus Crohn), chronischer unspezifischer Colitis und Divertikulitis sowie Verwendung von Salicylazobenzoesäure, zur Herstellung von derartigen Mitteln.**

(30) Priorität: **17.07.80 DE 3027013**

(43) Veröffentlichungstag der Anmeldung:
**03.02.82 Patentblatt 82/5**

(45) Bekanntmachung des Hinweises auf die Patenterteilung:
**18.04.84 Patentblatt 84/16**

(84) Benannte Vertragsstaaten:
**AT BE CH DE FR GB IT LI LU NL SE**

(56) Entgegenhaltungen:
**US - A - 2 482 752**

**CHEMICAL ABSTRACTS, Band 64, Nr. 3, 31. Januar 1966, Spalte 3403d, Columbus, Ohio, U.S.A., D.P. MITRA et al.: "5-Phenylazo and 5-tolylazosalicyclic acid derivatives and their antifungal and antibacterial activity"**
**MARTIN NEGWER "Organisch-chemische Arzneimittel und ihre Synonyma", 5. Auflage, Akademie-Verlag 1978, Band II, Seiten 640-641 Berlin, DE.**

(73) Patentinhaber: **Henning Berlin GmbH Chemie und Pharmawerk, Komturstrasse 19-20, D-1000 Berlin 42 (DE)**

(72) Erfinder: **Rokos, Hartmut, Dr., Am Fischtal 24b, D-1000 Berlin 37 (DE)**

(74) Vertreter: **Werner, Hans-Karsten, Dr. et al, Deichmannhaus am Hauptbahnhof, D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Mittel zur Behandlung von *Colitis ulcerosa, Enteritis regionalis* Crohn (Morbus Crohn), chronischer unspezifischer Kolitis und Divertikulitis sowie Verwendung von Salicylazobenzoesäure, zur Herstellung von derartigen Mitteln

Gegenstand der vorliegenden Erfindung ist ein neues Mittel zur Behandlung von *Colitis ulcerosa, Enteritis regionalis* Crohn (Morbus Crohn), chronischer unspezifischer Kolitis und Divertikulitis, enthaltend als Wirkstoff Salicylazobenzoesäure und/oder ihre physiologisch verträglichen Salze sowie übliche Hilfsstoffe. Weiterhin betrifft die Erfindung die Verwendung der Salicylazobenzoesäure und/oder ihre physiologisch verträglichen Salze zur Herstellung von Mitteln zur Behandlung dieser Erkrankungen.

Das Standardpräparat zur Behandlung dieser Krankheiten ist bisher Salazosulfapyridin. Diese Verbindung wird meist oral verabreicht und wahrscheinlich im Dickdarm bakteriell in die beiden Metaboliten 5-Aminosalicylsäure und Sulfapyridin gespalten. Anscheinend ist die wirksame Komponente der Metabolit 5-Aminosalicylsäure, während die häufig beobachteten Nebenwirkungen auf den Metaboliten Sulfapyridin zurückzuführen sind; vgl. Khan et al. „Lancet 2", 892 (1977). Da 5-Aminosalicylsäure instabil ist und auf oralem Weg praktisch nicht bis in den Dickdarm transportiert werden kann, wurde bereits von Khan, loc. cit. die Möglichkeit erwogen, einen neue Substanz zu synthetisieren, die diese Nachteile des bekannten Salazosulfapyridins nicht aufweist.

Es wurde jetzt gefunden, dass Salicylazobenzoesäure und/oder ihre physiologisch verträglichen Salze ausgezeichnet geeignet sind, das Salazosulfapyridin zu ersetzen. Es wurde weiter gefunden, dass die Spaltung der Salicylazobenzoesäure durch Darmbakterien etwa doppelt so schnell erfolgt wie die des Salazosulfapyridins, so dass der wirksame Metabolit 5-Aminosalicylsäure sehr rasch an der gewünschten Stelle freigesetzt wird. Der zweite Metabolit, 4-Aminobenzoesäure, ist offensichtlich weniger toxisch als Sulfapyridin und führt somit nicht zu den bekannten Nebenwirkungen des Salazosulfapyridins. Salicylazobenzoesäure wird vergleichbar dem Salazosulfapyridin im Dünndarm praktisch nicht resorbiert, so dass es unverändert bis in den Dickdarm mit seiner Bakterienflora transportiert wird.

Salicylazobenzoesäure ist eine bekannte Substanz, die als Farbstoff zur analytischen Bestimmung von Magnesium, Aluminium und Beryllium verwendet wurde, vgl. I. M. Korenman, E. I. Levina, „Azo Dyes-Derivates of Chromotropic and Salicylic Acids as the Reagents for Magnesium", Uchenye Zapiski Gor'kovsk. Gosudarst. Univ. im. Ni. I. Lobachevskogo, „Ser. Khim.", 1958, Nr. 32, 149-160. N. Appala Raju, K. Neelakantam, „Azo Dyes as Analytical Reagents for Aluminium and Beryllium. Current Sci." (India) 29, 52-3 (1960).

Trotz des Bekanntseins der Substanz an sich war nicht vorherzusehen, dass sie ausgezeichnet geeignet ist als Mittel zur Behandlung von *Colitis ulcerosa, Enteritis regionalis* Crohn (Morbus Crohn), chronischer unspezifischer Kolitis und Divertikulitis. Erst durch die jetzt durchgeführten Untersuchungen wurde festgestellt, dass Salicylazobenzoesäure in analoger Weise durch Darmbakterien in den Wirkstoff Aminosalicylsäure und einen untoxischen und unwirksamen Metaboliten 4-Aminobenzoesäure gespalten wird. Weiterhin wurde erst jetzt gefunden, dass diese Spaltung etwa doppelt so schnell verläuft wie die des bekannten Wirkstoffes Salazosulfapyridin. Keinesfalls vorhersehbar war, dass auch diese relativ niedermolekular Substanz im Dünndarm praktisch nicht resorbiert wird und daher nur lokal im Dickdarm und Enddarm zur Wirkung kommen kann. Dies ist besonders überraschend, da die Löslichkeit der Salicylazobenzoesäure in Wasser höher ist als die des Salazosulfapyridins. Die höhere Wasserlöslichkeit hingegen erweist sich als weiterer Vorteil bei der Herstellung von Arzneimitteln, da beispielsweise bei der Herstellung von Klysmen wesentlich höhere Konzentrationen/Volumeneinheit Klysma eingeführt werden können und bei der Herstellung von Tabletten und Dragées der Wirkstoffgehalt/Gewichtseinheit Tablette oder Dragée sehr hoch gewählt werden kann, ohne dass dadurch die Auflösung der Tablette im Darmtrakt beeinträchtigt wird.

Da die Spaltung der erfindungsgemäss verwendeten Salicylazobenzoesäure sowieso erst im Dickdarm und Enddarm erfolgen soll, sind die erfindungsgemässen Tabletten und Dragées vorzugsweise dünndarmlöslich. Hierzu wählt man z.B. eine Rezeptur, bei der die Stoffe nur im alkalischen Milieu des Dünndarms aufgelöst werden. Einfacher erreicht man dieses Ziel jedoch durch Überziehen der Tabletten oder Dragées mit einem dünndarmlöslichen Film.

Die Salicylazobenzoesäure kann erfindungsgemäss nicht nur in freier Form, sondern auch in Form ihrer physiologisch verträglichen Salze verwendet werden. Vorzugsweise geeignet sind die Alkali-, Erdalkali- und Ammoniumsalze. Prinzipiell können aber auch alle anderen, relativ leicht wasserlöslichen Salze physiologisch verträglicher Basen verwendet werden. Insbesondere bei der Herstellung von Klysmen kann es vorteilhaft sein, ein Gemisch der freien Salicylazobenzoesäure mit einem Salz zu verwenden, um den pH-Wert zu optimieren. Aber auch die oralen Zubereitungen können ganz oder teilweise die kristallinen Salze der Salicylazobenzoesäure anstelle der freien Säure enthalten.

Bei der Herstellung der erfindungsgemässen Mittel können alle üblichen Hilfsstoffe zur Herstellung von Tabletten und Dragées zur Anwendung kommen. Zu diesen üblichen Hilfsstoffen zählen Talkum, Stärke, Zellulose, Polyvinylpyrrolidon, Polyvinylpolypyrrolidon, Magnesiumstearat etc. Für die Herstellung von dünndarmlöslichen Rezepturen eignen sich insbesondere Polycarbonsäuren und andere Polymere, die nur im Alkalischen gespalten oder gelöst werden. Auch die üblichen dünndarmlöslichen Filmüberzüge bestehen meist aus derartigen sauren Polymeren, die erst im alkalischen Milieu des Dünndarms gelöst oder gespalten werden.

Die erfindungsgemässen Tabletten oder Dragées enthalten im allgemeinen 0,3 bis 1 g Salicylazobenzoesäure. Für die Behandlung akuter entzündlicher Prozesse werden Erwachsenen täglich 1 bis 6 g Salicylazobenzoesäure oral verabreicht. Zur Rezidivprophylaxe in der Dauertherapie reichen 1 bis 4 g mehreren Einzeldosen über den Tag verteilt. Da die bisher beobachteten Nebenwirkungen bei der Therapie mit Salazosulfapyridin bei den erfindungsgemässen Mitteln nicht beobachtet werden, kann erfindungsgemäss auch höher als bisher dosiert werden.

Die Therapie erfolgt im allgemeinen oral, wobei bevorzugt dünndarmlösliche Tabletten oder Filmdragées zur Anwendung kommen. Unter besonderen Umständen, insbesondere wenn die Verweilzeit im Darm stark reduziert ist, können auch magenlösliche Tabletten und Dragées zur Anwendung kommen.

Insbesondere bei stationärer Behandlung kommt anstelle der oralen Therapie auch die Einführung von Klysmen in Frage, wobei ein- oder mehrmals pro Tag bis zu 100 ml Klysma eingeführt werden können. Ein derartiges Klysma sollte 1 bis 6, vorzugsweise 3 g Salicylazobenzoesäure in 100 ml Lösung enthalten. Vorbereitete Klysmen sollten somit 10 bis 60, vorzugsweise 30 g Salicylazobenzoesäure/l Klysma enthalten.

Toxizitätsuntersuchungen der Salicylazobenzoesäure auch im Vergleich zum bisher verwendeten Salazosulfapyridin haben folgendes ergeben:

Im Ames-Test konnte keine mutagene Wirkung der Salicylazobenzoesäure festgestellt werden.

Die Salicylazobenzoesäure ist nach repetitiver rektaler Verabreichung am Kaninchendarm verträglich. Es wurden keine primären Hautreizungen und keine primären Schleimhautreizungen festgestellt. Die akute Toxizitätsprüfung nach oraler Applikation an der Ratte bis zu 10 g/kg ergab keine Mortalitäten, keine Hinweise auf Unverträglichkeiten und keine Abweichungen von Normalbefunden. Die Untersuchung der subakuten Toxizität an der Ratte ergab eine *no effect dosis* von 500 mg/kg.

Die Untersuchung der Resorption der Salicylazobenzoesäure in der abgebundenen Darmschlinge des Jejunum der Ratte ergab, dass die Substanz im Dünndarm nicht resorbiert wird, im Gegensatz zu anderen Azoderivaten der Salicylsäure.

Die Azospaltung der Salicylazobenzoesäure in einer Kotsuspension ergab, dass die Substanz bereits nach 60 min nicht mehr nachweisbar ist, während Salazosulfapyridin erst nach 120 min nicht mehr nachweisbar ist. Die Halbwertzeiten der Azospaltung betrugen bei der Salicylazobenzoesäure nur 15 min, während sie beim Salazosulfapyridin 27 min betragen.

Aus diesen Untersuchungen der Resorption und Spaltung geht eindeutig hervor, dass Salicylazobenzoesäure einen erheblich günstigeren therapeutischen Index aufweist als das bisher verwendete Salazosulfapyridin.

Die Herstellung der Salicylazobenzoesäure erfolgt nach an sich bekannten Methoden durch Diazotierung von p-Aminobenzoesäure und Kupplung mit Salicylsäure. Eine typische Herstellungsweise sowie typische Beispiele für die Herstellung erfindungsgemässer Mittel gehen aus den folgenden Beispielen hervor.

*Beispiel 1:*

*Herstellung der Salicylazobenzoesäure*

230 g 4-Aminobenzoesäure werden in 1 200 ml Wasser und 1 kg Eis suspendiert und mit 410 ml konzentrierter Salzsäure versetzt. Unter zusätzlicher äusserer Kühlung werden 117 g Natriumnitrit, gelöst in 700 ml Wasser, unter Rühren langsam zugegeben; die Temperatur wird dabei zwischen −5 und 0°C gehalten.

240 g Salicylsäure werden in 4500 ml kalter 2 mol/l Natronlauge gelöst. Unter starker äusserer Kühlung und starkem Rühren wird obige Diazoniumsalzlösung zugegeben; die Temperatur sollte dabei ca. 0°C betragen. Nach vollständiger Zugabe der Diazoniumlösung wird ohne weitere Kühlung noch 2 h nachgerührt.

Die Salicylazobenzoesäure wird durch langsame Zugabe verdünnter Salzsäure ausgefällt, bis der pH-Wert der Lösung ca. 2,5 beträgt. Der Niederschlag wird abgesaugt und gut mit Wasser gewaschen. 423 g, Ausbeute 87%. Zur weiteren Reinigung wird das Produkt in verdünntem Ammoniak gelöst, mit Aktivkohle behandelt, abgesaugt und wieder mit verdünnter Salzsäure ausgefällt.

*Analyse:* $C_{14}H_{10}N_2O_5$

| | | | |
|---|---|---|---|
| Berechnet: | C 58,74 | H 3,52 | O 27,94% |
| Gefunden: | C 58,62 | H 3,64 | O 27,67% |

*Beispiel 2:*

*Filmdragées*

1. *Zusammensetzung pro Kern*

500 mg Salicylazobenzoesäure (100%ig, wasserfrei)
  50 mg Cellulosepulver, DAB 8
  20 mg Polyvinylpolypyrrolidon
  20 mg Polyvinylpyrrolidon
  10 mg Mg-Stearat
_____
600 mg

2. *Herstellung*

50 kg Salicylazobenzoesäure
  5 kg Cellulosepulver
  1 kg Polyvinylpolypyrrolidon
  2 kg Polyvinylpyrrolidon

werden homogen gemischt. Unter Zusatz von gereinigtem Wasser (EAB) wird granuliert und bei 50°C getrocknet.

  1 kg Polyvinylpolypyrrolidon
  1 kg Mg-Stearat

werden untergemischt.

Es wird zu Dragéekernen von 600 mg Gewicht verpresst.

Die Kerne werden wahlweise mit einem üblichen Filmcoating-Verfahren mit einem dünndarmlöslichen oder magenlöslichen Film überzogen.

*Beispiel 3:*

*Klysma*

Zur Herstellung eines Klysma werden in 1000 ml Wasser gelöst bzw. suspendiert:

| | |
|---|---|
| 30 g | Salicylazobenzoesäure |
| 9 g | Natriumchlorid |
| 5 g | Carbopol 934® (mit Natronlauge neutralisiert) (Carboxyvinylpolymer) |
| 2 g | Methylcellulose 25 (wasserlöslicher Celluloseäther) |
| 1 g | Tween 80® (Polyoxyäthylensorbetanmonooleat) |
| 0,5 g | Ascorbinsäure |
| 0,4 g | Benzalkoniumchlorid (50%ige Lösung) (Benzyldimethylalkylammoniumchlorid) |

Jeweils 100 ml werden in Flaschen abgefüllt und verschlossen.

*Beispiel 4:*

*Klysma*

Zur Herstellung eines Klysma werden in ca. 800 ml Wasser gelöst bzw. suspendiert:

| | |
|---|---|
| 30 g | Salicylazobenzoesäure |
| 5 g | Carbopol 934® (Carboxyvinylpolymer) |
| 2 g | Methylcellulose 25 (wasserlöslicher Celluloseäther) |
| 1 g | Tween 80® (Polyoxyäthylensorbetanmonooleat) |
| 0,5 g | Ascorbinsäure |
| 0,4 g | Benzalkoniumchlorid (50%ige Lösung) (Benzyldimethylalkylammoniumchlorid) |

Die Mischung wird mit Natronlauge neutralisiert (pH ca. 7,3), wobei eine klare Lösung entsteht, die mit Wasser auf 1000 ml aufgefüllt wird.

Jeweils 100 ml werden in Flaschen abgefüllt und verschlossen.

## Patentansprüche

1. Mittel zur Behandlung von *Colitis ulcerosa, Enteritis regionalis* Crohn (Morbus Crohn), chronischer unspezifischer Kolitis und Divertikulitis enthaltend Salicylazobenzoesäure und/oder ihre physiologisch verträglichen Salze sowie übliche Hilfsstoffe.

2. Mittel gemäss Anspruch 1, dadurch gekennzeichnet, dass sie 0,3 bis 1 g Salicylazobenzoesäure und/oder ihre physiologisch verträglichen Salze in Form von Tabletten oder Dragées enthalten.

3. Mittel gemäss den Ansprüchen 1 und 2, dadurch gekennzeichnet, dass die Tabletten oder Dragées dünndarmlöslich sind.

4. Mittel gemäss Anspruch 1, dadurch gekennzeichnet, dass es 10 bis 60 g Salicylazobenzoesäure und/oder ihre physiologisch verträglichen Salze in gelöster oder suspendierter Form/l Klysma enthält.

5. Verwendung von Salicylazobenzoesäure und/oder ihre physiologisch verträglichen Salze zur Herstellung von Mitteln zur Behandlung von *Colitis ulcerosa, Enteritis regionalis* Crohn (Morbus Crohn), chronischer unspezifischer Kolitis und Divertikulitis.

6. Verwendung gemäss Anspruch 5 zur Herstellung von Tabletten oder Dragées, enthaltend 0,3 bis 1 g Salicylazobenzoesäure und/oder ihre physiologisch verträglichen Salze.

7. Verwendung gemäss den Ansprüchen 5 und 6 zur Herstellung von dünndarmlöslichen Tabletten oder Dragées, enthaltend 0,3 bis 1 g Salicylazobenzoesäure und/oder ihre physiologisch verträglichen Salze.

8. Verwendung gemäss Anspruch 5 zur Herstellung von Klysmen, enthaltend 10 bis 60 g gelöster oder suspendierter Salicylazobenzoesäure und/oder ihre physiologisch verträglichen Salze/l Klysma.

## Claims

1. An agent for the treatment of *Colitis ulcerosa, Enteritis regionalis* Crohn (Morbus Crohn), chronic unspecifical colitis and diverticulitis, containing salicylic azobenzoic acid and/or the physiologically acceptable salts thereof and conventional adjuvants.

2. The agents according to Claim 1, characterized in that they contain 0.3 to 1 g of salicylic azobenzoic acid and/or the physiologically acceptable salts thereof in the form of tablets or dragées.

3. The agents according to Claims 1 and 2, characterized in that the tablets or dragées are soluble in the small intestine.

4. The agents according to Claim 1, characterized in that it contains 10 to 60 g of salicylic azobenzoic acid and/or the physiologically acceptable salts thereof in the dissolved or suspended form/l of clysma.

5. Use of salicylic azobenzoic acid and/or the physiologically acceptable salts thereof for the preparation of agents for the treatment of *Colitis ulcerosa, Enteritis regionalis* Crohn (Morbus Crohn), chronic unspecifical colitis and diverticulitis.

6. Use according to Claim 5 for the preparation of tablets or dragées containing 0.3 to 1 g of salicylic azobenzoic acid and/or the physiologically acceptable salts thereof.

7. Use according to Claims 5 and 6 for the preparation of tablets or dragées which are soluble in the small intestine and contain 0.3 to 1 g of salicylic azobenzoic acid and/or the physiologically acceptable salts thereof.

8. Use according to Claim 5 for the preparation of clysma liquids containing 10 to 60 g of dissolved or suspended salicylic azobenzoic acid and/or the physiologically acceptable salts thereof/l of clysma.

## Revendications

1. Médicament pour le traitement de la *Colitis ulcerosa*, de l'*Enteritis regionalis* Crohn (Morbus Crohn), d'une colite chronique non spécifique et de la diverticulite, contenant de l'acide salicylazobenzoïque et/ou ses sels physiologiquement acceptables, ainsi que les adjuvants usuels.

2. Médicament selon la revendication 1, caractérisé en ce qu'ils contiennent 0,3 à 1 g d'acide salicylazobenzoïque et/ou de ses sels physiologiquement acceptables, sous forme de comprimés ou de dragées.

3. Médicament selon les revendications 1 et 2, caractérisé en ce que les comprimés ou dragées sont solubles dans l'intestin grêle.

4. Médicament selon la revendication 1, caractérisé en ce qu'il contient 10 à 60 g d'acide salicylazobenzoïque et/ou de ses sels physiologiquement acceptables, sous forme dissoute ou en suspension, par litre de lavement.

5. Utilisation de l'acide salicylazobenzoïque et/ou de ses sels physiologiquement acceptables pour préparer des produits pour le traitement de la *Colitis ulcerosa*, de l'*Enteritis regionalis* Crohn (Morbus Crohn), d'une colite chronique non spécifique et de la diverticulite.

6. Utilisation selon la revendication 5 pour la préparation de comprimés ou de dragées contenant 0,3 à 1 g d'acide salicylazobenzoïque et/ou de ses sels physiologiquement acceptables.

7. Utilisation selon les revendications 5 et 6 pour la préparation de comprimés ou de dragées solubles dans l'intestin grêle, contenant 0,3 à 1 g d'acide salicylazobenzoïque et/ou de ses sels physiologiquement acceptables.

8. Utilisation selon la revendication 5 pour la préparation de lavements contenant 10 à 60 g d'acide salicylazobenzoïque et/ou de ses sels physiologiquement compatibles, dissous ou en suspension, par litre de lavement.